# EUROPEAN PATENT APPLICATION

(11) **EP 4 847 506 A1**
(43) Date of publication of application: **02.12.2026**
(21) Application number: 25179337.8
(22) Date of filing: 28.05.2025
(51) Int. Cl.: C07D 417/04, C07C 311/21

(54) **PROCESS FOR THE PREPARATION OF DABRAFENIB**

(71) Applicant: Midas Pharma GmbH, 55218 Ingelheim (DE)
(72) Inventor: IVÁNYI, Zoltán, 1104 Budapest (HU); FARKAS, Ferenc, 2119 Pécel (HU)
(74) Representative: Drescher, Christian

(57) **Abstract**

The present invention provides a process for the preparation of dabrafenib or any pharmaceutically acceptable salt, ester, solvate or derivative thereof comprising one or more of the following steps:
i. crystallizing methyl 3-{[(2,6-difluorophenyl)sulfonyl]amino}-2-fluorobenzoate from 2-propanol,
ii. reacting N-{3-[(2-chloro-4-pyrimidinyl)acetyl]-2-fluorophenyl}-2,6-difluorobenzene sulfonamide with N-bromosuccinimide and subsequently with 2,2-Dimethylpropanethioamide in ethyl acetate,
iii. reacting N-{3-[5-(2-chloro-4-pyrimidinyl)-2-(1,1-dimethylethyl)-1,3-thiazol-4-yl]-2-fluorophenyl}-2,6-difluorobenzene-sulfonamide with ammonia solution at temperatures of from 78 to 85°C and crystallizing the resulting N-{3-[5-(2-amino-4-pyrimidinyl)-2-(1,1-dimethylethyl)-1,3-thiazol-4-yl]-2-fluorophenyl}-2,6-difluorobenzenesulfonamide (Dabrafenib) from n-heptane at temperatures of from 15 to 25°C,
iv. reacting N-{3-[5-(2-amino-4-pyrimidinyl)-2-(1,1-dimethylethyl)-1,3-thiazol-4-yl]- 2-fluorophenyl}-2,6-difluorobenzenesulfonamide (dabrafenib) with methanesulfonic acid in methyl ethyl ketone to form dabrafenib mesylate.

## Description

### BACKGROUND OF THE INVENTION

Dabrafenib (also known as N-{3-[5-(2-amino-4-pyrimidinyl)-2-(1,1-dimethylethyl)-1,3-thiazol-4-yl]-2-fluorophenyl}-2,6-difluorobenzenesulfonamide) in the form of its mesylate salt is currently registered and marketed under the brand name TAFINLAR^{®} for
- the treatment of unresectable or metastatic melanoma with a BRAF V600 mutation in adult patients with as monotherapy or in combination with trametinib,
- the adjuvant treatment of adult patients with Stage III melanoma with a BRAF V600 mutation, following complete resection in combination with trametinib, and
- the treatment of adult patients with advanced non-small cell lung cancer with a BRAF V600 mutation in combination with trametinib.

Dabrafenib and dabrafenib mesylate may be prepared by a 5-step process according to WO 2009/137391 (Examples 58 c and d):

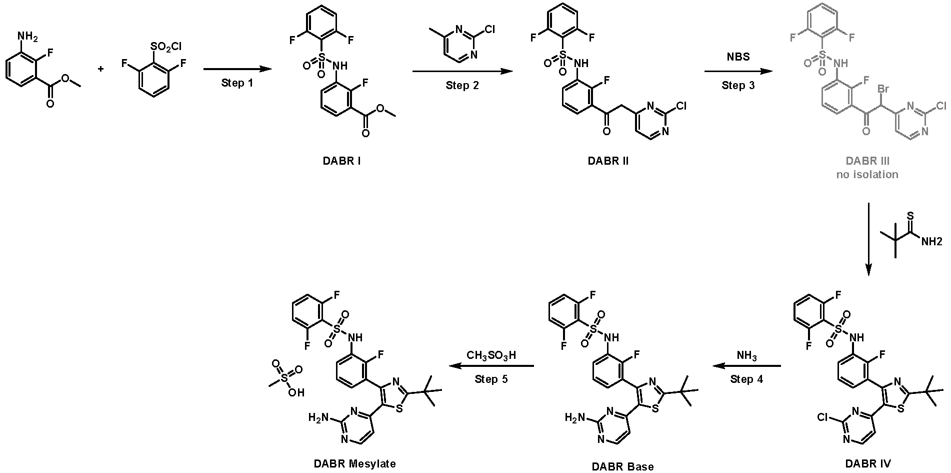

The route of synthesis according to WO 2009/137391 has several drawbacks:
• Step 1 involves the workup of DABR I in ethyl acetate which requires several aqueous washing steps and the use of hazardous cyclohexane as an anti-solvent for the DABR I crystallization.
• Step 3 involves a complicated solvent exchange from dichloromethane to ethyl acetate between the initial reaction of DABR II with N-bromosuccinimide (NBS) and the subsequent reaction of DABR III with Dimethylpropanethioamide (DMPTA)
• Step 4 involves the reaction of DABR IV with ammonium hydroxide at high temperatures of up to more than 100°C which leads to the formation of high amounts of the undesired and potentially mutagenic by-product N-{3-[5-(2-amino-4-pyrimidinyl)-2-(1,1-dimethylethyl)-1,3-thiazol-4-yl]-2-fluorophenyl}-(2-Amino-6-fluorobenzene)sulfonamide (desfluoroamino impurity) which is difficult to remove.

| | |
|---|---|
| N-{3-[5-(2-amino-4-pyrimidinyl)-2-(1,1-dimethylethyl)-1,3-thiazol-4-yl]-2-fluorophenyl}-(2-Amino-6-fluorobenzene)sulfonamide (desfluoroamino impurity) | 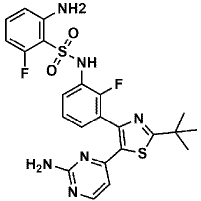 |

• Step 5 involves the solvent isopropanol which is very difficult to remove from the crystalline product and has poor purging properties with regards to the undesired by-product N-{3-[5-(2-amino-4-pyrimidinyl)-2-(1,1-dimethylethyl)-1,3-thiazol-4-yl]-2-fluorophenyl}-(2-Amino-6-fluorobenzene)sulfonamide (desfluoroamino impurity).

There is a need to develop an improved method for industrial large-scale synthesis of dabrafenib and dabrafenib mesylate.

### DESCRIPTION OF THE INVENTION

The present invention provides an improved process for the preparation of dabrafenib and dabrafenib mesylate that overcomes the disadvantages of the preparation methods in the prior art. The preparation method of the present invention is simple in operation, high in capacity, simple in equipment, safe, low in cost and suitable for scale-up, i.e. for industrialized production.

In a first aspect, the present invention provides an improved process for the crystallization of methyl 3-{[(2,6-difluorophenyl)sulfonyl]amino}-2-fluorobenzoate (DABR I) from isopropanol. By using isopropanol instead of ethyl acetate, DABR I can be crystallized directly from the solvent which simplifies the work-up procedure significantly. In addition, the use of the hazardous anti-solvent cyclohexane can be avoided and the yield as well as the purity of DABR I are improved.

The present invention is thus directed to a process of crystallizing methyl 3-{[(2,6-difluorophenyl)sulfonyl]amino}-2-fluorobenzoate (DABR I) from 2-propanol.

The present invention also provides an improved process of the preparation of dabrafenib or any pharmaceutically acceptable salt, ester, solvate or derivative thereof comprising the step of crystallization of the intermediate methyl 3-{[(2,6-difluorophenyl)sulfonyl]amino}-2-fluorobenzoate (DABR I) from 2-propanol.

Furthermore, the present invention provides a process for the preparation of dabrafenib or any pharmaceutically acceptable salt, ester, solvate or derivative thereof comprising the steps of
(i) reacting methyl 3-amino-2-fluorobenzoate (MeAFB) with 2,6-diflurorobenzenesulfonyl chloride (DiFSCl) in a solvent in the presence of a base,
(ii) exchanging the solvent with isopropyl alcohol
(iii) crystallizing the reaction product methyl 3-{[(2,6-difluorophenyl)sulfonyl]amino}-2-fluorobenzoate (DABR I) from 2-propanol.

The preferred solvent is methylene chloride and the preferred base is pyridine.

In a more preferred embodiment, process step (ii) comprises the following steps:
(ii-a) adding 4 to 8 equivalents (w/w relative to MeAFB) of 2-propanol,
(ii-b) concentrating the reaction mixture to about 40 % volume,
(ii-c) adding 4 to 8 equivalents (w/w relative to MeAFB) of 2-propanol.

In a second aspect, the present invention provides a process of reacting N-{3-[(2-chloro-4-pyrimidinyl)acetyl]-2-fluorophenyl}-2,6-difluorobenzenesulfonamide (DABR II) with N-bromosuccinimide (NBS) in ethyl acetate to obtain N-{3-[(2-chloro-4-pyrimidinyl)-1-Bromoacetyl]-2-fluorophenyl}-2,6-difluorobenzene-sulfonamide (DABR III) which is reacted in the same solvent with 2,2-Dimethylpropanethioamide (DMPTA) to obtain N-{3-[5-(2-chloro-4-pyrimidinyl)-2-(1,1-dimethylethyl)-1,3-thiazol-4-yl]-2-fluorophenyl}-2,6-difluorobenzenesulfonamide (DABR IV).

Carrying out both the initial bromination and the subsequent formation of the thiazole ring in the same solvent renders a solvent exchange obsolete and the intermediate DABR III does not need to be isolated. In addition, the use of hazardous dichloromethane can be avoided.

The present invention is thus directed to a process of reacting N-{3-[(2-chloro-4-pyrimidinyl)acetyl]-2-fluorophenyl}-2,6-difluorobenzene sulfonamide (DABR II) with N-bromosuccinimide and subsequently reacting the intermediate N-{3-[(2-chloro-4-pyrimidinyl)-1-Bromo-acetyl]-2-fluorophenyl}-2,6-difluorobenzene-sulfonamide with 2,2-Dimethylpropanethioamide in ethyl acetate to obtain N-{3-[5-(2-chloro-4-pyrimidinyl)-2-(1,1-dimethylethyl)-1,3-thiazol-4-yl]-2-fluorophenyl}-2,6-difluorobenzenesulfonamide (DABR IV). In a preferred embodiment, N-{3-[(2-chloro-4-pyrimidinyl)-1-Bromo-acetyl]-2-fluorophenyl}-2,6-difluoro benzene-sulfonamide (DABR III) is not isolated.

The present invention also provides an improved process for the preparation of N-{3-[5-(2-chloro-4-pyrimidinyl)-2-(1,1-dimethylethyl)-1,3-thiazol-4-yl]-2-fluorophenyl}-2,6-difluorobenzenesulfonamide (DABR IV) by reacting N-{3-[(2-chloro-4-pyrimidinyl)acetyl]-2-fluorophenyl}-2,6-difluorobenzene sulfonamide (DABR II) with N-bromosuccinimide and subsequently with 2,2-Dimethylpropanethioamide in ethyl acetate.

In addition, the present invention provides an improved process for the preparation of dabrafenib comprising the steps of
(i) reacting N-{3-[(2-chloro-4-pyrimidinyl)acetyl]-2-fluorophenyl}-2,6-difluorobenzene-sulfonamide (DABR II) with N-bromosuccinimide in ethyl acetate,
(ii) reacting the intermediate N-{3-[(2-chloro-4-pyrimidinyl)-1-Bromo-acetyl]-2-fluorophenyl}-2,6-difluorobenzene-sulfonamide (DABR III) with 2,2-Dimethylpropanethioamide (DMPTA) in ethyl acetate.

In a preferred embodiment, N-{3-[(2-chloro-4-pyrimidinyl)-1-Bromo-acetyl]-2-fluorophenyl}-2,6-difluoro benzene-sulfonamide (DABR III) is not isolated.

In a preferred embodiment, the process comprises the additional step (iii) of
(1) adding of water to extract the dimethyl acetamide and inorganic materials,
(2) crystallizing the reaction product N-{3-[5-(2-chloro-4-pyrimidinyl)-2-(1,1-dimethylethyl)-1,3-thiazol-4-yl]-2-fluorophenyl}-2,6-difluorobenzenesulfonamide (DABR IV) from a mixture of ethyl acetate and n-heptane.

In a third aspect, the present invention provides an improved process of reacting N-{3-[5-(2-chloro-4-pyrimidinyl)-2-(1,1-dimethylethyl)-1,3-thiazol-4-yl]-2-fluorophenyl}-2,6-difluorobenzene-sulfonamide (DABR IV) with ammonia solution at much lower temperatures of from 78 to 85 °C to obtain N-{3-[5-(2-amino-4-pyrimidinyl)-2-(1,1-dimethylethyl)-1,3-thiazol-4-yl]-2-fluorophenyl}-2,6-difluorobenzenesulfonamide (DABR Base, Dabrafenib). By this means the formation of the potentially mutagenic by-product N-{3-[5-(2-amino-4-pyrimidinyl)-2-(1,1-dimethylethyl)-1,3-thiazol-4-yl]-2-fluorophenyl}-(2-Amino-6-fluorobenzene)sulfonamide (desfluoroamino impurity), which is critical because it is very hard to remove, can be significantly reduced compared to the process disclosed in the prior art.

In addition, the crystallization of N-{3-[5-(2-amino-4-pyrimidinyl)-2-(1,1-dimethylethyl)-1,3-thiazol-4-yl]-2-fluorophenyl}-2,6-difluorobenzenesulfonamide (DABR Base) from n-heptane can be performed at room temperature which saves time and energy.

Accordingly, the present invention provides a process for the preparation of N-{3-[5-(2-amino-4-pyrimidinyl)-2-(1,1-dimethylethyl)-1,3-thiazol-4-yl]-2-fluorophenyl}-2,6-difluorobenzenesulfonamide (dabrafenib, DABR Base) or a pharmaceutically acceptable salt, ester, solvate or derivative thereof by reacting N-{3-[5-(2-chloro-4-pyrimidinyl)-2-(1,1-dimethylethyl)-1,3-thiazol-4-yl]-2-fluorophenyl}-2,6-difluorobenzene-sulfonamide (DABR IV) with ammonia solution at temperatures of from 78 to 85°C and crystallizing the resulting DABR Base from n-heptane at temperatures of from 15 to 25°C.

The present invention also provides a process for the preparation of dabrafenib or a pharmaceutically acceptable salt, ester, solvate or derivative thereof comprising the step of
(i) reacting N-{3-[5-(2-chloro-4-pyrimidinyl)-2-(1,1-dimethylethyl)-1,3-thiazol-4-yl]-2-fluorophenyl}-2,6-difluorobenzene-sulfonamide (DABR IV) with aqueous ammonia solution at temperatures of from 78 to 85 °C.

In preferred embodiments, the process comprises one or more of the additional steps of
(i-a) adding ethyl acetate to the reaction and extracting the organic phase with water, and/or
(ii) crystallizing of N-{3-[5-(2-amino-4-pyrimidinyl)-2-(1,1-dimethylethyl)-1,3-thiazol-4-yl]-2-fluorophenyl}-2,6-difluorobenzenesulfonamide (DABR Base) from n-heptane at temperatures of from 15 to 25 °C.

In a fourth aspect, the present invention provides a process of reacting N-{3-[5-(2-amino-4-pyrimidinyl)-2-(1,1-dimethylethyl)-1,3-thiazol-4-yl]-2-fluorophenyl}-2,6-difluorobenzenesulfonamide (dabrafenib) with methanesulfonic acid in methyl ethyl ketone to obtain the methanesulfonic acid salt of N-{3-[5-(2-amino-4-pyrimidinyl)-2-(1,1-dimethylethyl)-1,3-thiazol-4-yl]-2-fluorophenyl}-2,6-difluorobenzenesulfonamide (dabrafenib mesylate, DABR Mesylate). Since isopropanol is very hard to remove from the crystalline product, the switch from isopropanol to methyl ethyl ketone results in a lower residual solvent content. In addition, methyl ethyl ketone allows a more efficient purge of the critical desfluoroamino impurity. Finally, the use of highly flammable diethyl ether can also be avoided.

The present invention is thus directed to a process of reacting N-{3-[5-(2-amino-4-pyrimidinyl)-2-(1,1-dimethylethyl)-1,3-thiazol-4-yl]-2-fluorophenyl}-2,6-difluorobenzenesulfonamide (DABR Base) with methanesulfonic acid in methyl ethyl ketone.

In addition, the present invention provides an improved process for the preparation of dabrafenib mesylate comprising the reaction step of
(i) reacting N-{3-[5-(2-amino-4-pyrimidinyl)-2-(1,1-dimethylethyl)-1,3-thiazol-4-yl]-2-fluorophenyl}-2,6-difluorobenzenesulfonamide (DABR Base) with methanesulfonic acid in methyl ethyl ketone,
(ii) removing the methyl ethyl ketone by suspending the crude product in a mixture of ethyl acetate and water and evaporating methyl ethyl ketone and ethyl acetate.

In a preferred embodiment, the reaction of dabrafenib with methanesulfonic acid in methyl ethyl ketone in step (i) is conducted at temperatures of from 20°C to 30°C, preferably 23 to 27 °C.

Preferably, the ratio of the mixture of ethyl acetate and water is from 5:1 to 7:1 (w/w), more preferred 6:1 (w/w).

As already mentioned above, the formation of N-{3-[5-(2-amino-4-pyrimidinyl)-2-(1,1-dimethylethyl)-1,3-thiazol-4-yl]-2-fluorophenyl}-(2-Amino-6-fluorobenzene)sulfonamide (Desfluoroamino impurity) is critical because it is very difficult to remove. Since various attempts to remove the desfluoroamino impurity on commercial scale via crystallization, derivatization column chromatography etc. failed, the formation of this impurity must be avoided by optimization of its route of synthesis. After many attempts, the formation of the desfluoroamino impurity could be significantly reduced by two different means, i.e.
(iii) reduction of the temperature in the amination step (Step 4) to 78-85 °C according to example 4 of the present invention
(iv) replacement of the solvent isopropanol by methyl ethyl ketone in the salt formation step (step 5) of the present invention

As displayed in table 1, the synthesis of dabrafenib according to example 58c step D of WO 2009/137391 leads to 1.75% of desfluoroamino impurity. By conversion into dabrafenib Mesylate according to the process of Example 58d, this impurity could not be reduced to less than 1.5%.

On the other hand, the process according to example 4 of the present invention results in dabrafenib with a much lower level of 0.24% of desfluoroamino impurity.

In addition, the high level of 1.75% of desfluoroamino impurity could be reduced to not more than 0.08% by employing the process according to example 5 of the present invention.

Combining examples 4 and 5 of the present invention results in dabrafenib mesylate with a level of typically less than 0.05% of desfluoroamino impurity.

**Table 1**

| **Compound** | **Process** | **Desfluoroamino impurity** | **Limit** |
|---|---|---|---|
| dabrafenib | WO 2009/137391, Example 58c step D | 1.75 % | 0.5 |
| | Present invention example 4 | 0.24 % | |
| dabrafenib mesylate | WO 2009/137391, Example 58c Step D + Example 58d | 1.5 % | 0.15 |
| | WO 2009/137391, Example 58c Step D + example 5 | 0.08 % | |
| | Present invention example 4 + 5 | ≤ 0.05 % | |

Accordingly, the present invention is directed to a process of providing N-{3-[5-(2-amino-4-pyrimidinyl)-2-(1,1-dimethylethyl)-1,3-thiazol-4-yl]-2-fluorophenyl}-2,6-difluorobenzenesulfonamide (dabrafenib) with an amount of N-{3-[5-(2-amino-4-pyrimidinyl)-2-(1,1-dimethylethyl)-1,3-thiazol-4-yl]-2-fluorophenyl}-(2-Amino-6-fluorobenzene) sulfonamide (desfluoroamino impurity) of less than 1.75% w/w, preferably not more than 0.5% w/w.

In addition, the present invention is directed to a process of providing N-{3-[5-(2-amino-4-pyrimidinyl)-2-(1,1-dimethylethyl)-1,3-thiazol-4-yl]-2-fluorophenyl}-2,6-difluorobenzenesulfonamide (dabrafenib mesylate) with a content of N-{3-[5-(2-amino-4-pyrimidinyl)-2-(1,1-dimethylethyl)-1,3-thiazol-4-yl]-2-fluorophenyl}-(2-Amino-6-fluorobenzene)sulfonamide of less than 1.5% w/w, preferably not more than 0.1% w/w.

The present invention also provides a process for the preparation of dabrafenib or any pharmaceutically acceptable salt, ester, solvate or derivative thereof comprising one or more of the following steps:
i. crystallizing methyl 3-{[(2,6-difluorophenyl)sulfonyl]amino}-2-fluorobenzoate from 2-propanol,
ii. reacting N-{3-[(2-chloro-4-pyrimidinyl)acetyl]-2-fluorophenyl}-2,6-difluorobenzene sulfonamide with N-bromosuccinimide and subsequently with 2,2-Dimethylpropanethioamide in ethyl acetate to obtain N-{3-[5-(2-chloro-4-pyrimidinyl)-2-(1,1-dimethylethyl)-1,3-thiazol-4-yl]-2-fluorophenyl}-2,6-difluorobenzenesulfonamide,
iii. reacting N-{3-[5-(2-chloro-4-pyrimidinyl)-2-(1,1-dimethylethyl)-1,3-thiazol-4-yl]-2-fluorophenyl}-2,6-difluorobenzene-sulfonamide with ammonia solution at temperatures of from 78 to 85°C and crystallizing the resulting N-{3-[5-(2-amino-4-pyrimidinyl)-2-(1,1-dimethylethyl)-1,3-thiazol-4-yl]-2-fluorophenyl}-2,6-difluorobenzenesulfonamide (dabrafenib) from a mixture of ethyl acetate and n-heptane at temperatures of from 15 to 25°C
iv. reacting N-{3-[5-(2-amino-4-pyrimidinyl)-2-(1,1-dimethylethyl)-1,3-thiazol-4-yl]- 2-fluorophenyl}-2,6-difluorobenzenesulfonamide (dabrafenib) with methanesulfonic acid in methyl ethyl ketone to form dabrafenib mesylate.

### EXAMPLES

### Example 1: Preparation of Methyl 3-{[(2,6-difluorophenyl)sulfonyl]amino}-2-fluorobenzoate (DABR I)

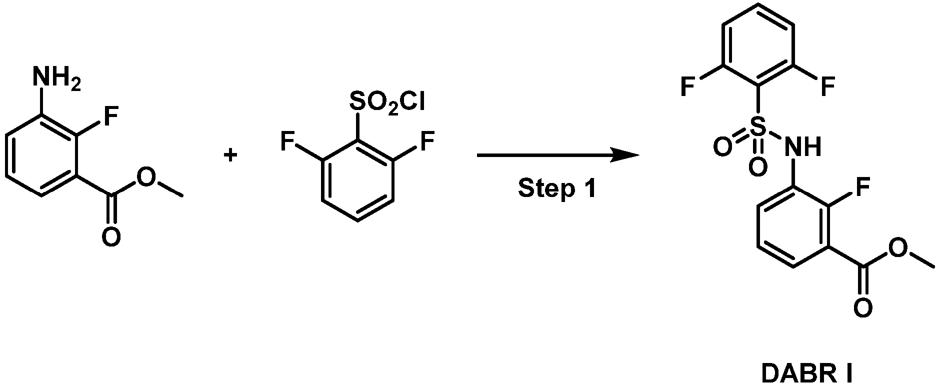

124.8 kg Methylene chloride and 20.8 kg previously melted methyl 3-amino-2-fluorobenzoate (MeAFB) are charged into the reactor, mixed until complete dissolution and cooled to 10 °C. 10.7 kg Pyridine is then added to the reactor at 8 to 12 °C. 27.4 kg of 2,6-diflurorobenzenesulfonyl chloride (DiFSCl) is added to the mixture at 10 to 20 °C. The reaction mixture is stirred for 10-12 hours at 20 to 25 °C and then concentrated to about 60 L (ca. 40% volume). 122.5 kg isopropyl alcohol is added to the residue and the mixture is again concentrated to about 60 L. Again, 122.5 kg isopropyl alcohol is added to the residue and the mixture is heated to 20 to 25 °C and stirred until crystallization is completed. The crystals are filtered and washed with isopropyl alcohol. The wet product is dried under vacuum at 47 to 53 °C for 8-12 hours to obtain 40 kg (94%) of methyl 3-{[(2,6-difluorophenyl)sulfonyl]amino}-2-fluorobenzoate (DABR I).

### Example 2: Preparation of N-{3-[(2-chloro-4-pyrimidinyl)acetyl]-2-fluorophenyl}-2,6-difluorobenzene sulfonamide (DABR II)

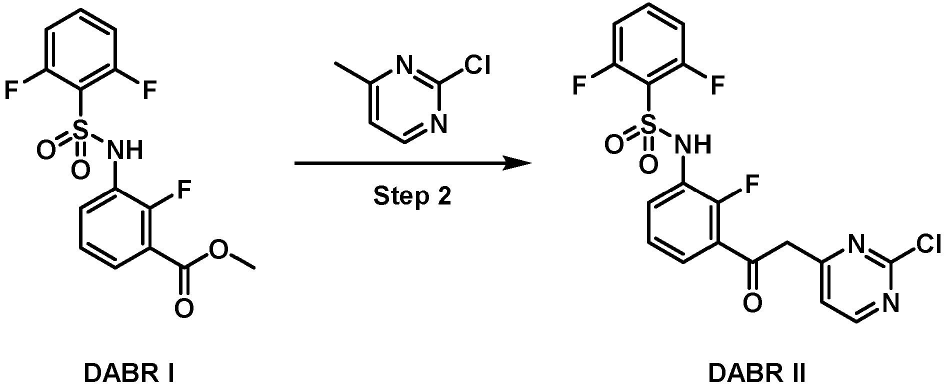

29.0 kg methyl 3-{[(2,6-difluoro-phenyl)sulfonyl]amino}-2-fluorobenzoate (DABR I) and 128.5 kg Tetrahydrofuran (anhydrous) are loaded into a reactor and stirred until dissolution. The mixture is cooled to 0 to 5 °C and 290 kg Lithium bis(trimethylsilyl)amide (20% solution in THF) is added at -5 to +5 °C. A solution of 14 kg 2-chloro-4-methylpyrimidine (ClMePyr) in 64.4 kg Tetrahydrofuran (anhydrous) is added at -5 to +5 °C and stirred at 0 to 5 °C for 60-90 minutes. The mixture is heated up to 20 to 25 °C and stirred for 60-90 minutes. The mixture is cooled to 0 to 10 °C and 222 kg of aqueous hydrochloric acid solution (1:1 w/w) is added to the mixture at 0 to 10 °C. After acid addition the mixture is heated up to 15 to 20 °C and the pH is adjusted to a value of 1-2 with HCl. The aqueous phase is separated and the organic phase is reduced by vacuum distillation to about half volume. 236 kg of isopropyl acetate and 138 liters of water are added. After the aqueous phase is separated, the extraction step is repeated. After washing, the organic phase is reduced by vacuum distillation to approx. one third of volume. 158 kg of isopropyl acetate is loaded into the reactor and the reaction mixture is evaporated again to approx. half volume. 145 kg Methyl tert-butyl ether is added and the mixture is stirred at 20 to 25 °C for at least 1 hour. The resulting crystals are filtered and rinsed with methyl tert-butyl ether. The wet product is dried in vacuum at 45 to 55 °C for 8-12 hours to obtain 30.4 6kg (82%) of N-{3-[(2-chloro-4-pyrimidinyl)acetyl]-2-fluorophenyl}-2,6-difluorobenzene-sulfonamide (DABR II).

### Example 3: Preparation of N-{3-[5-(2-chloro-4-pyrimidinyl)-2-(1,1-dimethylethyl)-1,3-thiazol-4-yl]- 2-fluorophenyl}-2,6-difluorobenzenesulfonamide (DABR IV)

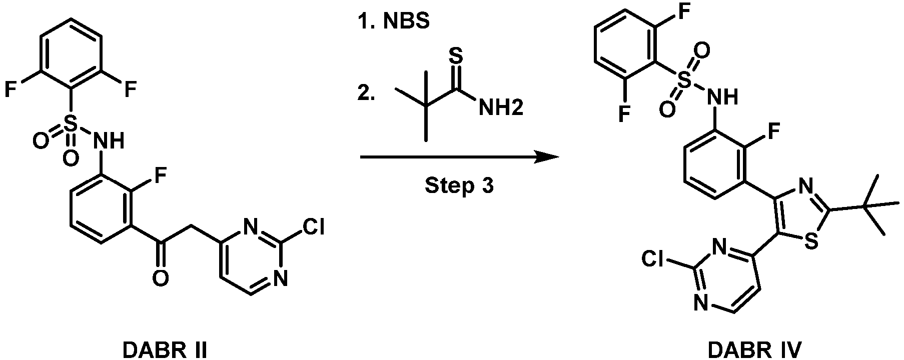

29.7 kg N-{3-[(2-chloro-4-pyrimidinyl)acetyl]-2-fluorophenyl}-2,6-difluorobenzenesulfonamide (DABR II) and 80.2 kg ethyl acetate are charged into a reactor, and the mixture is cooled to 0 to 5 °C. 12 kg n-Bromosuccinimide are added in several portions to the reaction mixture at 0 to 5 °C and the reaction mixture is heated to 20 to 25 °C. 125.5 kg dimethyl acetamide is added and the reaction mixture is cooled to 5 to 10 °C. A solution of 7.9 kg of 2,2- dimethylpropanethioamide (DMPTA) in 71.4 kg dimethyl acetamide is added to the reaction mixture while the temperature is maintained at 5 to 10 °C. After complete addition, the mixture is heated to 20 to 25 °C and stirred for 45-50 minutes at this temperature. Then the mixture is further heated to 70 to 75 °C and stirred this temperature for 2 hours. The mixture is then cooled to 8 to 12 °C and 208 kg water are added while the temperature is maintained below 30 °C. 178 kg ethyl acetate (anhydrous) is added to the reaction mixture and the phases are separated. The aqueous phase is extracted two times with 92 kg ethyl acetate (anhydrous). The combined organic phases are washed with water. The organic phase is concentrated by vacuum distillation to about one third of residual volume. 106.8 kg Ethyl acetate is added, and distillation is repeated to about half residual volume. The vacuum is released with nitrogen. Temperature of the concentrate is adjusted to 50 to 55 °C and 81.2 kg n-heptane is added while the temperature is maintained above 50 °C. The mixture is heated to 70 to 75 °C, then cooled to 0 to 5 °C in 60-150 minutes. The mixture is kept at 0 to 5 °C for 2-3 hours for crystallization. The resulting suspension is filtered on a centrifuge and the solid residue is washed with 27 kg n- heptane. The wet product is dried in vacuum at 45 to 55 °C for 4-8 hours to obtain 25.7 kg (71%) of N-{3-[5-(2-chloro-4-pyrimidinyl)-2-(1,1-dimethylethyl)-1,3-thiazol-4-yl]-2-fluorophenyl}-2,6-difluorobenzene-sulfonamide (DABR IV).

### Example 4: Preparation of N-{3-[5-(2-amino-4-pyrimidinyl)-2-(1,1-dimethylethyl)-1,3-thiazol-4-yl]- 2-fluorophenyl}-2,6-difluorobenzenesulfonamide (DABR Base)

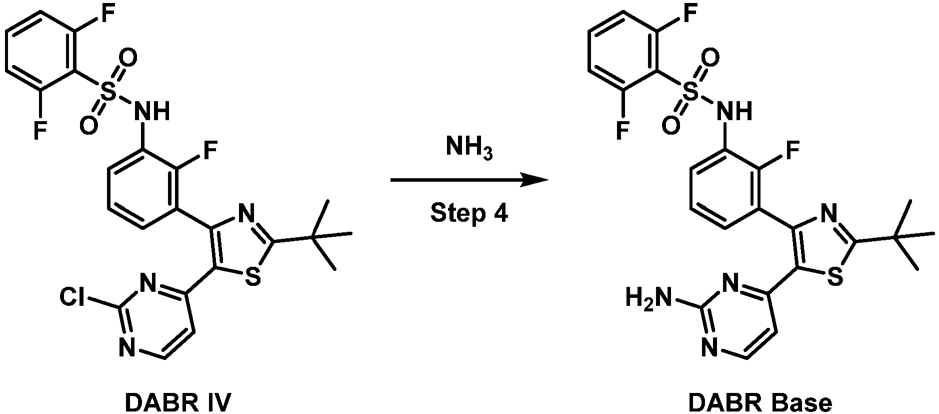

19.2 kg N-{3-[5-(2-chloro-4-pyrimidinyl)-2-(1,1-dimethylethyl)-1,3-thiazol-4-yl]-2-fluorophenyl}-2,6-difluorobenzenesulfonamide (DABR IV) and 350 kg of 24% aqueous ammonia solution are charged into a pressure reactor under inert atmosphere. The mixture is heated to 78 to 85 °C and stirred at this temperature for 8-9 hours. After cooling to 20 to 25 °C, the suspension is filtered and the resulting crystals are washed with water. 260 kg Ethyl acetate, 65 kg water and the wet product are charged into the reactor. The suspension is stirred until complete dissolution at 65 to 70 °C, then the aqueous phase is separated. Water is added to the organic phase and hydrochloric acid solution is added to the solution until pH 5-6 is reached. The mixture is heated to 65 to 70 °C and stirred for 15-20 minutes, then the aqueous phase is separated. 65 kg water is added to the organic phase, and the extraction step (heating, settling, phase separation) is repeated. The organic phase is cooled to 20 to 25 °C and concentrated to about one third of volume by vacuum distillation. 104 kg ethyl acetate is added to the concentrated organic phase and evaporation is repeated to about half volume. The concentrated organic phase is heated to 65 to 70 °C and stirred for 25-35 mins. 118.2 kg n-heptane is added to the reactor at this temperature. The resulting suspension is stirred for 2-3 hours at 65 to 70 °C, then cooled to 20 to 25 °C and stirred for at least 30 minutes. The crystal suspension is filtered on a centrifuge and the filter cake is washed with 44,7 kg n-heptane. The wet product is dried in vacuum for 3-5 hours at 27 to 33 °C and then for 3-5 hours at 47 to 53 °C to obtain 15.7 kg (85%) of N-{3-[5-(2-amino-4-pyrimidinyl)-2-(1,1-dimethylethyl)-1,3-thiazol-4-yl]-2-fluorophenyl}-2,6-difluorobenzenesulfonamide (dabrafenib, DABR Base).

### Example 5: Preparation of DABR Mesylate

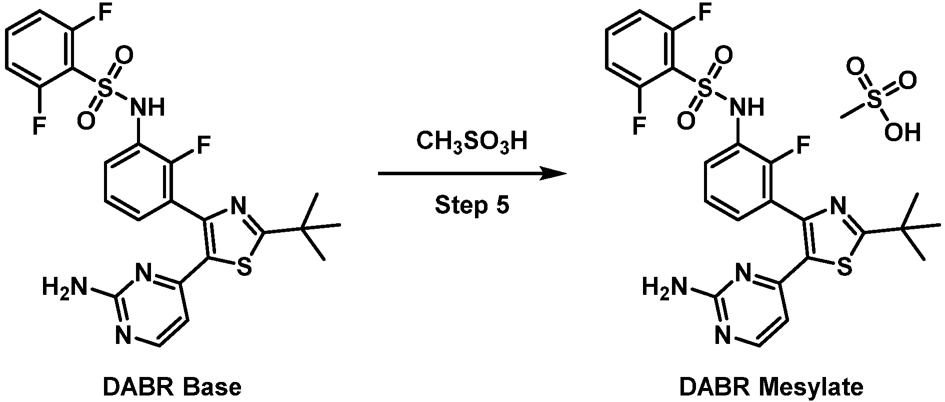

340 kg methyl ethyl ketone and 22.1 kg N-{3-[5-(2-amino-4-pyrimidinyl)-2-(1,1-dimethylethyl)-1,3- thiazol-4-yl]-2-fluorophenyl}-2,6-difluorobenzenesulfonamide (DABR Base) are mixed until dissolved and then filtered. The filter is washed with methyl ethyl ketone. 7 kg water was added to the filtrate at 23 to 27 °C. The solution was seeded with 0.22 kg dabrafenib mesylate and stirred for 5-10 minutes. 19.5 kg Methanesulfonic acid is added to the mixture during at 23 to 27 °C. If the seeding crystals dissolve, the solution is seeded again with dabrafenib mesylate 15 minutes after the start point of methanesulfonic acid addition. The suspension is stirred at 23 to 27 °C for 16-18 hours. The crystal suspension is filtered on a centrifuge and the filter cake is washed with three times 44.5 kg methyl ethyl ketone. The crude product is suspended in 199 kg ethyl acetate and 33 kg water, heated to reflux temperature and stirred at this temperature for 10-12 hours. One third of the liquid is distilled off at atmospheric pressure, and 66.3 kg ethyl acetate are added. Distillation and addition of ethyl acetate are repeated 3 more times. The resulting suspension is cooled to 20 to 25 °C and stirred at this temperature until crystallization is complete. The crystal suspension is filtered and the filter cake is washed with 46 kg ethyl acetate twice. The wet product is dried in vacuum at 20 to 26 °C for 4-8 hours to obtain 22 kg (84%) of N-{3-[5-(2-amino-4-pyrimidinyl)-2-(1,1-dimethylethyl)-1,3-thiazol-4-yl]-2-fluorophenyl}-2,6-difluorobenzenesulfonamide mesylate (dabrafenib mesylate, DABR Mesylate).

## Claims

1. A process for the preparation of dabrafenib or any pharmaceutically acceptable salt, ester, solvate or derivative thereof comprising the reaction steps of
(i) reacting methyl 3-amino-2-fluorobenzoate (MeAFB) with 2,6-diflurorobenzenesulfonyl chloride (DiFSCl) in a solvent in the presence of a base,
(ii) exchanging the solvent with 2-propanol,
(iii) crystallizing the reaction product methyl 3-{[(2,6-difluorophenyl)sulfonyl]amino}-2-fluorobenzoate from 2-propanol.

2. The process according to claim 1 wherein the solvent in step (i) is methylene chloride.

3. The process according to claim 1 or 2 wherein the base in step (i) is pyridine.

4. The process according any of the preceding claims, wherein step (ii) comprises the steps of
(ii-a) adding 4 to 8 equivalents (w/w relative to MeAFB) of 2-propanol,
(ii-b) concentrating the reaction mixture to about 40 % volume,
(ii-c) adding 4 to 8 equivalents (w/w relative to MeAFB) of 2-propanol.

5. A process for the preparation of dabrafenib or any pharmaceutically acceptable salt, ester, solvate or derivative thereof comprising the steps of
(i) reacting N-{3-[(2-chloro-4-pyrimidinyl)acetyl]-2-fluorophenyl}-2,6-difluorobenzene-sulfonamide with N-bromosuccinimide in ethyl acetate,
(ii) reacting the intermediate N-{3-[(2-chloro-4-pyrimidinyl)-1-Bromo-acetyl]-2-fluorophenyl}-2,6-difluorobenzene-sulfonamide with 2,2-Dimethylpropanethioamide (DMPTA) in ethyl acetate.

6. The process according to claim 5 **characterized in that** the intermediate N-{3-[(2-chloro-4-pyrimidinyl)-1-Bromo-acetyl]-2-fluorophenyl}-2,6-difluorobenzene-sulfonamide is not isolated.

7. A process according to claim 5 or 6 comprising the additional steps of
(iii) adding water to extract the dimethyl acetamide and inorganic materials,
(iv) crystallizing the reaction product N-{3-[5-(2-chloro-4-pyrimidinyl)-2-(1,1-dimethylethyl)-1,3-thiazol-4-yl]-2-fluorophenyl}-2,6-difluorobenzenesulfonamide from a mixture of ethyl acetate and n-heptane.

8. A process for the preparation dabrafenib or any pharmaceutically acceptable salt, ester, solvate or derivative thereof comprising the step of
(i) reacting N-{3-[5-(2-chloro-4-pyrimidinyl)-2-(1,1-dimethylethyl)-1,3-thiazol-4-yl]-2-fluorophenyl}-2,6-difluorobenzene-sulfonamide with aqueous ammonia solution at temperatures of from 78 to 85 °C.

9. The process according to claim 8, further comprising the additional step (ii) of crystallizing N-{3-[5-(2-amino-4-pyrimidinyl)-2-(1,1-dimethylethyl)-1,3-thiazol-4-yl]-2-fluorophenyl}-2,6-difluorobenzenesulfonamide (dabrafenib) from n-heptane at temperatures of from 15 to 25 °C.

10. The process according to claim 8 or 9, further comprising the additional step (i-a) of adding ethyl acetate to the reaction and extracting the organic phase with water.

11. A process for the preparation of dabrafenib according to any of the preceding claims wherein the content of N-{3-[5-(2-amino-4-pyrimidinyl)-2-(1,1-dimethylethyl)-1,3-thiazol-4-yl]-2-fluorophenyl}-(2-Amino-6-fluorobenzene)sulfonamide is less than 1.75% w/w.

12. A process for the preparation of dabrafenib mesylate comprising the reaction steps of
(i) reacting N-{3-[5-(2-amino-4-pyrimidinyl)-2-(1,1-dimethylethyl)-1,3-thiazol-4-yl]-2-fluorophenyl}-2,6-difluorobenzenesulfonamide (dabrafenib) with methanesulfonic acid in methyl ethyl ketone,
(ii) removing the methyl ethyl ketone by suspending the crude product in a mixture of ethyl acetate and water and evaporating methyl ethyl ketone and ethyl acetate.

13. The process according to claim 12 wherein the reaction of dabrafenib with methanesulfonic acid in methyl ethyl ketone is carried out at temperatures of from 20 to 30 °C.

14. The process according to claim 12 or 13 wherein the ratio of the mixture of ethyl acetate and water is from 5:1 to 7:1 w/w.

15. A process for the preparation of dabrafenib mesylate according to any of the preceding claims wherein the content of N-{3-[5-(2-amino-4-pyrimidinyl)-2-(1,1-dimethylethyl)-1,3-thiazol-4-yl]-2-fluorophenyl}-(2-Amino-6-fluorobenzene)sulfonamide is less than 1.5% w/w.
